# EUROPEAN PATENT APPLICATION

(11) **EP 4 197 650 A1**
(43) Date of publication of application: **21.06.2023**
(21) Application number: 21215419.9
(22) Date of filing: 17.12.2021
(51) Int. Cl.: B05C 5/02, A61F 13/15, B65H 57/04, D06B 11/00

(54) **A UNIT FOR APPLYING GLUE ON CONTINUOUS ELEMENTS AND RELATED METHOD**

(71) Applicant: Fameccanica.Data S.p.A., 66020 San Giovanni Teatino (CH) (IT)
(72) Inventor: BONELLI, Guido, I-66020 San Giovanni Teatino (Chieti) (IT); LUSI, Luca, I-66020 San Giovanni Teatino (Chieti) (IT)
(74) Representative: Marchitelli, Mauro

(57) **Abstract**

A glue application unit (10) comprising a glue dispenser (18), a feeding device (12) for feeding at least one continuous elongated element (14) in contact with a terminal portion of a nozzle assembly (22) of the glue application unit (10), and a detachment element (32) for detaching the at least one continuous elongated element (14) from the nozzle assembly (22) when the feeding device (12) is stopped,

## Description

### Field of the invention

The present invention relates to a unit for applying glue on continuous elongated elements moving in a longitudinal direction.

The invention was developed, in particular, with a view to its application in the field of manufacturing absorbent sanitary articles, such as diapers, training pants, female sanitary napkins, etc.

In the following description, reference will be made to this specific field without however losing generality.

One aspect of the invention relates to a method for applying glue to continuous elongated elements.

### Description of the prior art

In the field of manufacturing absorbent sanitary articles there is often the need of applying glue on continuous elongated elements. For example, for manufacturing elastic waist bands of absorbent sanitary articles a plurality of longitudinally tensioned elastic threads parallel to one another are attached by glue between two non-woven webs, as disclosed for instance in EP-A-2186493. Glue is usually applied intermittently on the tensioned elastic threads. In other applications glue may be applied on a web, e.g. a non-woven web or a plastic film, with a pattern which defines the areas in which the web has to be attached to other components of the absorbent sanitary articles.

The glue used in these applications is typically a hot melt glue applied by a heated glue dispenser. Hot melt glue is solid at room temperature and has to be heated at about 150-200 °C for being dispensed in fluid state.

The movable elongated elements, e.g. elastic threads or webs, on which the hot melt glue has to be applied are usually in contact with the delivery nozzles of the glue dispenser.

When the glue dispensing unit is stopped, the continuous elongated elements remain in contact with the nozzles of the glue dispenser for a time much longer than the contact time expected during normal operation.

Even if heating of the glue dispenser is interrupted when the unit is stopped, the thermal inertia of the glue dispenser maintains a high temperature of the nozzles for a long period of time.

Prolonged contact of a portion of the elongated elements with the hot surface of the nozzles may cause severe damages. If the elements are made of non-woven webs or plastic, excessive heating may damage or perforate the webs. If the elongated elements are elastic threads excessive heating can cause the plastic material to melt. The hot glue present on the elongated elements may melt and may contaminate some parts of the equipment.

In order to solve these problems, it has been proposed to move the feeding rollers which feed the continuous elongated elements to the glue dispenser, when the unit is stopped. The feeding rollers may be moved to a position in which the continuous elongated elements are detached from the glue dispenser.

This solution is not always feasible, especially when glue has to be applied on a multitude of tensioned elastic threads (in some cases even 100 parallel threads). In such cases, movement of the feeding rollers may cause losing of the alignment between the elastic threads and the nozzles of the glue dispenser, which would lead to long down times.

Given the complexity and the poor results of the prior art solutions, many customers prefer not to have any detachment system during machine stops, thus encountering various problems, such as contamination or damages of the threads or webs.

### Object and summary of the invention

The object of the present invention is to provide a glue application unit which overcomes the problems of the prior art.

According to the present invention, this object is achieved by a glue application unit having the features of claim 1.

According to another aspect, the present invention relates to a converting machine comprising a such a glue application unit and to a method for applying glue on continuous elongated elements, having the features of claim 12.

The claims form an integral part of the disclosure provided here in relation to the invention.

### Brief description of the drawings

The present invention will now be described in detail with reference to the attached drawings, given purely by way of non-limiting example, wherein:
- Figures 1 and 2 are schematic side views of a glue application unit according to an embodiment of the present invention, respectively in an operating position and in a stop position,
- Figures 3 and 4 are schematic front views respectively along the lines III and IV of figures 1 and 2, and
- Figures 5 and 6 are schematic side views of another embodiment of a glue application unit according to the present invention, respectively in an operating position and in a stop position.

### Detailed description

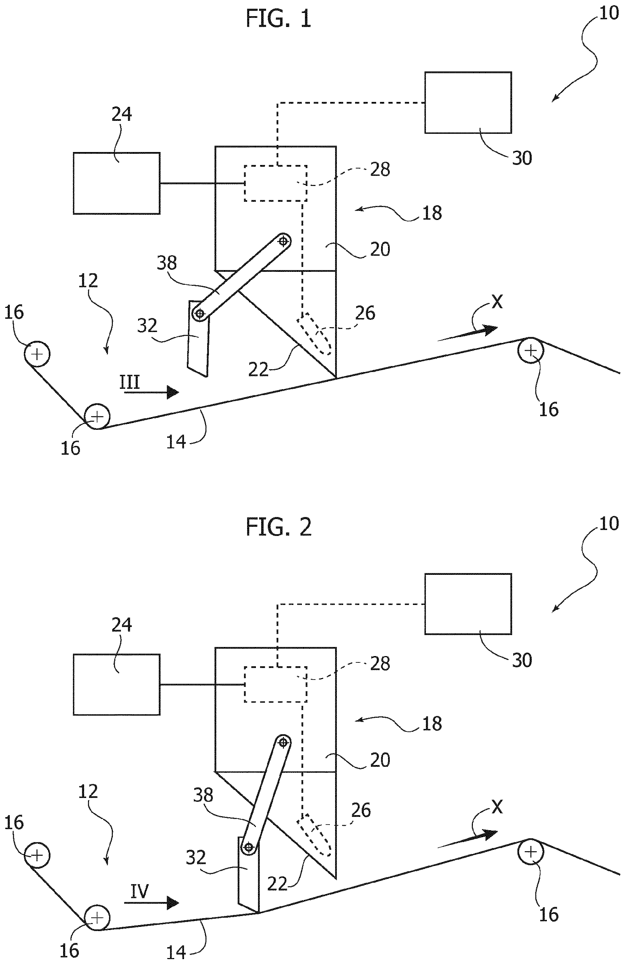

With reference to Figures 1 and 2, reference 10 indicates a glue application unit according to an embodiment of the present invention.

The glue application unit 10 comprises a feeding device 12 configured for feeding at least one continuous elongated element 14 in a longitudinal direction X. Figure 1 shows the glue application unit 10 in an operating position in which the feeding device 12 continuously advances in the longitudinal direction X the at least one continuous elongated element 14 and figure 2 shows the glue application unit 10 in a stop position in which the feeding device 12 is stopped and the at least one continuous elongated element 14 is stationary.

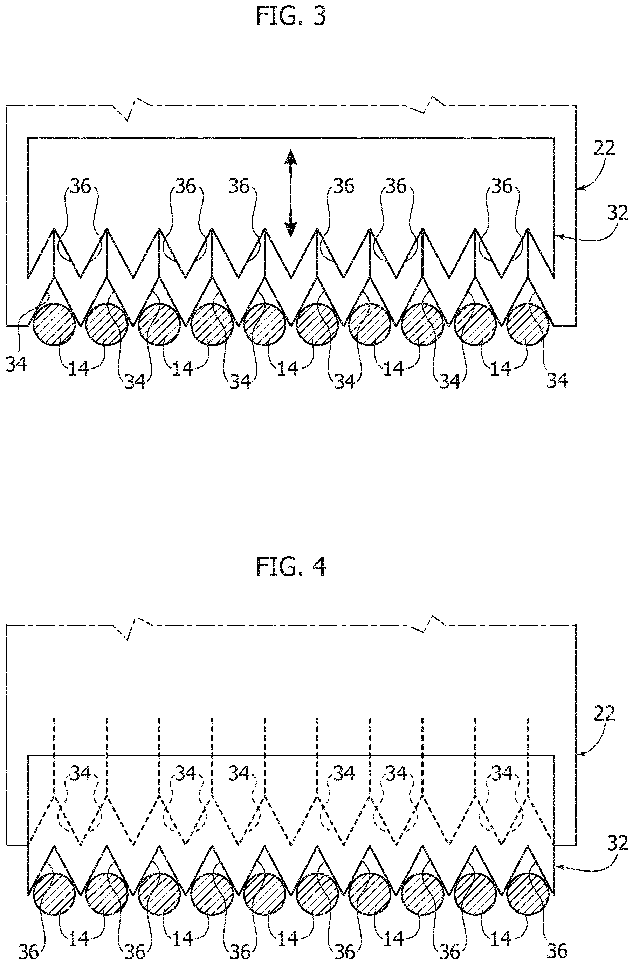

With reference to figures 3 and 4, in a possible embodiment, the feeding device 12 may be configured for feeding a plurality of continuous elastic threads 14 parallel to each other.

In another possible embodiment, the feeding device 12 may be configured for feeding a continuous elongated web 14, e. g. a non-woven web, a plastic film, a composite laminate, etc.

With reference to Figures 1 and 2, the feeding device 12 may comprise a plurality of guide rollers 16 which guide the at least one continuous elongated element 14 along a feeding path. The position of the guide rollers 16 may be adjustable to adjust the feeding path and/or the longitudinal tension of the at least one continuous elongated element 14, but the guide rollers 16 remain in the same position both when the glue application unit 10 is in the operating condition and when the glue application unit 10 is in a stop condition.

The glue application unit 10 comprises a glue dispenser 18 including a dispenser body 20 and a nozzle assembly 22 configured for dispensing hot melt glue in a fluid state on the continuously movable elongated element(s) 14.

The glue application unit 10 may comprise a glue reservoir 24 containing hot melt glue in a fluid state, at a temperature of about 150-200 °C. The glue reservoir 24 is fluidically connected to a plurality of nozzles 26 arranged in the nozzle assembly 22. The glue dispenser 18 may comprise a valve unit 28 which selectively turns on and off the fluid connection to the nozzles 26. The valve unit 28 may be actuated intermittently in order to apply glue intermittently on the continuously movable elongated element(s) 14. The valve unit 28 may be controlled by an electronic control unit 30 which selectively turns on and off the valve unit 28 in phase with the movement of the continuously movable elongated element(s) 14 in order to form a desired glue pattern on the elongated element(s) 14.

The glue application unit 10 comprises a detachment element 32 configured for detaching the continuous elongated element(s) 14 from the nozzle assembly 22 when the feeding device 12 is stopped.

The detachment element 32 is movable between a disengaging position corresponding to the operating condition of the glue application unit 10 and an engaging position corresponding to the stop condition of the glue application unit 10.

In the operating condition of the glue application unit 10, shown in figure 1, the at least one continuous elongated element 14 continuously moves in the longitudinal direction X along a feeding path which leads the at least one continuous elongated element 14 in contact with a terminal portion of the nozzle assembly 22 of the glue application unit 10. In the operating condition the detachment element 32 does not contact the at least one continuous elongated element 14, which is therefore free to follow the preset feeding path.

In the stop condition of the glue application unit 10, shown in figure 2, the detachment element 32 is in contact with the at least one continuous elongated element 14 and deviates the at least one continuous elongated element 14 from the preset path. When the detachment element 32 is in the engaging position the at least one continuous elongated element 14 is detached from the terminal portion of the nozzle assembly 22.

The detachment element 32 is in close proximity to the nozzle assembly 22. The close proximity between the detachment element 32 and the nozzle assembly 22 allows the continuous elongated element 14 to be kept stable in cross-direction in the engaging position. In a possible embodiment the distance between the detachment element 32 and the nozzle assembly 22 along the longitudinal direction X is comprised between 2 - 20 cm, preferably between 5 - 10 cm.

The movement of the detachment element 32 from the disengaging position to the engaging position may be controlled by the electronic control unit 30 in conjunction with a command that turns off the valve unit 28 when the feeding device 12 is stopped.

In a possible embodiment, the detachment element 32 may be carried by the dispenser body 20 of the glue dispenser 18. The detachment element 32 may be made of a thermally insulating material or may be connected to the dispenser body 20 through thermally insulating elements, so as not to transfer heat to the at least one continuous elongated element 14 when the detachment element 32 contacts the at least one continuous elongated element 14.

With reference to figures 3 and 4, when the glue application unit 10 is configured for applying glue on a plurality of continuous elastic threads 14 parallel to each other, the nozzle assembly 22 may comprise a plurality of first recesses 34 spaced apart from each other along a direction perpendicular to said longitudinal direction (X), each receiving a respective elastic thread 14. The detachment element 32 may comprise a plurality of second recesses 36, aligned to the first recesses 34 of the nozzle assembly 22, such that when the detachment element 32 is in the engaging position (figure 4) the alignment between the continuous elastic threads 14 and the first recesses 34 is maintained.

As shown in figures 1 and 2, the detachment element 32 is preferably arranged upstream of the nozzle assembly 22 with reference to the direction of movement of the continuous elastic element(s) 14, so that the detachment element 32 is not contaminated by glue when it contacts the continuous elastic element(s) 14.

The detachment element 32 may be plate-shaped and may be connected to the dispenser body 20 via a pair of pivoting arms 38.

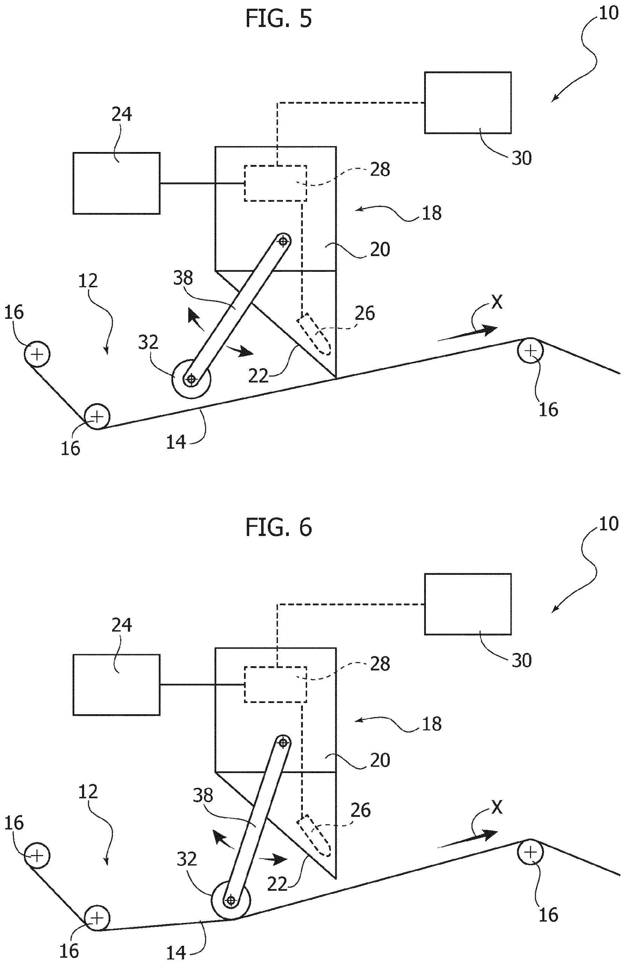

With reference to figures 5 and 6, in a possible embodiment the detachment element 32 may comprise a roller 40. The roller 40 may be freely rotatable about an axis of rotation orthogonal to the longitudinal direction X of the continuous elastic element(s) 14. The roller 40 may be arranged upstream of the nozzle assembly and may be connected to the dispenser body 20 via a pair of pivoting arms 38. The roller 40 may be made of thermally insulating material or may be connected to the dispenser body 20 via thermally insulating elements. The roller 40 may be smooth if the feeding device 12 is configured for feeding a continuous elongated web 14 or may be provided with a plurality of parallel annular grooves if the feeding device 12 is configured for feeding a plurality of continuous elastic threads 14 parallel to each other.

The glue application unit 10 previously disclosed may be incorporated in a converting machine, for instance in a machine for manufacturing absorbent sanitary articles.

Of course, without prejudice to the principle of the invention, the details of construction and the embodiments can be widely varied with respect to those described and illustrated, without thereby departing from the scope of the invention as defined by the claims that follow.

## Claims

1. A glue application unit (10) comprising:
- a glue dispenser (18) including a dispenser body (20) and a nozzle assembly (22) configured for dispensing hot melt glue in a fluid state on at least one continuously movable elongated element (14),
- a feeding device (12) configured for feeding at least one continuous elongated element (14) in a longitudinal direction (X) along a preset feeding path which leads the at least one continuous elongated element (14) in contact with a terminal portion of the nozzle assembly (22) of the glue application unit (10),
- a detachment element (32) configured for detaching the at least one continuous elongated element (14) from the nozzle assembly (22) when the feeding device (12) is stopped,
wherein the detachment element (32) is movable between a disengaging position in which it does not contact the at least one continuous elongated element (14) during an operating condition of the glue application unit (10) and an engaging position in which it contacts the at least one continuous elongated element (14) during a stop condition of the glue application unit (10) and deviates the at least one continuous elongated element (14) from said preset path.

2. The glue application unit of claim 1, wherein said feeding device (12) is configured for feeding a plurality of continuous elastic threads (14) parallel to each other.

3. The glue application unit of claim 2, wherein said detachment element (32) comprises a plurality of second recesses (36) configured for receiving respective continuous elastic threads (14), said second recesses being spaced apart from each other along a direction perpendicular to said longitudinal direction (X).

4. The glue application unit of claim 3, wherein the nozzle assembly (22) comprises a plurality of first recesses (34) configured for receiving respective continuous elastic threads (14) and aligned to respective second recesses (36) of said detachment element (32).

5. The glue application unit of any of the preceding claims, wherein the detachment element (32) is arranged upstream of the nozzle assembly (22).

6. The glue application unit of any of the preceding claims, wherein the detachment element (32) is plate shaped or comprises a roller (40) freely rotatable about an axis of rotation orthogonal to the longitudinal direction (X) of the at least one continuous elastic element (14).

7. The glue application unit of any of the preceding claims, wherein the detachment element (32) is carried by the dispenser body (20) of the glue dispenser (18).

8. The glue application unit of claim 7, wherein the detachment element (32) is made of thermally insulating material or is connected to the dispenser body (20) via thermally insulating elements.

9. The glue application unit of any of the preceding claims, wherein the distance between the detachment element (32) and the nozzle assembly (22) along said longitudinal direction (X) is comprised between 2 - 20 cm, preferably between 5 - 10 cm.

10. The glue application unit of any of the preceding claims, wherein the glue dispenser (18) comprises a valve unit (28) which selectively turns on and off the fluid connection to nozzles (26), wherein said valve unit (28) is controlled by an electronic control unit (30), and wherein the movement of the detachment element (32) from the disengaging position to the engaging position is controlled by said electronic control unit (30) in conjunction with a command that turns off the valve unit (28) when the feeding device (12) is stopped.

11. A converting machine comprising a glue application unit (10) according to any of the preceding claims.

12. A method for applying glue, comprising:
- providing a glue dispenser (18) including a dispenser body (20) and a nozzle assembly (22),
- feeding at least one continuous elongated element (14) in a longitudinal direction (X) along a preset feeding path which leads the at least one continuous elongated element (14) in contact with a terminal portion of the nozzle assembly (22) of the glue application unit (10),
- dispensing hot melt glue in a fluid state on said at least one continuously movable elongated element (14) at said nozzle assembly (22),
- providing a detachment element (32) movable between a disengaging position in which it does not contact the at least one continuous elongated element (14) during an operating condition of the glue application unit (10) and an engaging position in which it contacts the at least one continuous elongated element (14) during a stop condition of the glue application unit (10) and deviates the at least one continuous elongated element (14) from said preset path.

13. The method of claim 12, comprising feeding along said preset feeding path a plurality of continuous elastic threads (14) parallel to each other.

14. The method of claim 13, comprising feeding said plurality of continuous elastic threads (14) through respective first recesses (34) of said nozzle assembly (22) and engaging said plurality of continuous elastic threads (14) by respective second recesses (36) of said detachment element (32) aligned to said first recesses (34) of the nozzle assembly (22).

15. The method of any of claims 12-14, comprising providing a valve unit (28) which selectively turns on and off the fluid connection to nozzles (26), controlling said valve unit (28) by an electronic control unit (30), and controlling the movement of the detachment element (32) from the disengaging position to the engaging position by said electronic control unit (30) in conjunction with a command that turns off the valve unit (28) when the feeding device (12) is stopped.
